(19) Europäisches Patentamt
European Patent Office
Office européen des brevets

(11) EP 3 289 495 B1

(12) EUROPEAN PATENT SPECIFICATION

(45) Date of publication and mention of the grant of the patent:
**29.05.2024 Bulletin 2024/22**

(21) Application number: **16717742.7**

(22) Date of filing: **04.04.2016**

(51) International Patent Classification (IPC):
***G16C 20/30*** *(2019.01)*

(52) Cooperative Patent Classification (CPC):
**G16C 20/30**

(86) International application number:
**PCT/US2016/025929**

(87) International publication number:
**WO 2016/178763 (10.11.2016 Gazette 2016/45)**

# (54) METHOD TO REPRESENT METAL CONTENT IN CRUDE OILS, REACTOR FEEDSTOCKS, AND REACTOR PRODUCTS

VERFAHREN ZUR DARSTELLUNG DES METALLGEHALTS IN ROHÖLEN, REAKTORROHSTOFFE UND REAKTORPRODUKTE

PROCÉDÉ DE REPRÉSENTATION DE LA TENEUR EN MÉTAL DANS DES PÉTROLES BRUTS, CHARGES D’ALIMENTATION DE RÉACTEURS, ET PRODUITS DE RÉACTEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.05.2015 US 201562155791 P**
**18.02.2016 US 201615047347**

(43) Date of publication of application:
**07.03.2018 Bulletin 2018/10**

(73) Proprietor: **AspenTech Corporation**
**Bedford, MA 01730 (US)**

(72) Inventors:
- **WATANASIRI, Suphat**
  **Bedford ,MA 01730 (US)**
- **WANG, Shu**
  **Acton, MA 01720 (US)**
- **YU, Lili**
  **Shanghai 201208 (CN)**

(74) Representative: **Appleyard Lees IP LLP**
**15 Clare Road**
**Halifax HX1 2HY (GB)**

(56) References cited:
**EP-A1- 2 584 381 US-A1- 2013 185 044**

- **MCKENNA A. M. ET AL: "Unprecedented Ultrahigh Resolution FT-ICR Mass Spectrometry and Parts-Per-Billion Mass Accuracy Enable Direct Characterization of Nickel and Vanadyl Porphyrins in Petroleum from Natural Seeps", ENERGY & FUELS, vol. 28, no. 4, 17 April 2014 (2014-04-17) , pages 2454-2464, XP055210353, ISSN: 0887-0624, DOI: 10.1021/ef5002452**
- **G. SEBOR ET AL: "Effect of the type of organometallic iron and copper compounds on the determination of both metals in petroleum samples by flame atomic-absorption spectroscopy", THE ANALYST, vol. 107, no. 1280, 1 January 1982 (1982-01-01), pages 1350-1355, XP055276664, GB ISSN: 0003-2654, DOI: 10.1039/AN9820701350**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 289 495 B1

## Description

### BACKGROUND OF THE INVENTION

Each type of crude oil has unique molecular characteristics. These characteristics include different physical and chemical properties that can potentially affect the refining process. Typically, crude oils contain trace amounts of metal, such as vanadium or nickel. These metals become concentrated in the residual fractions of the refining process and can contaminate the products, poison catalysts, or corrode equipment. Refineries, therefore, seek to remove these metals prior to, or during, the refining process. Before a metal can be removed from crude oil, however, the refineries must identify the characteristics of the crude oil, including which metals are present and their concentrations. To do so, refineries can generate characterization data (or assay data) of a crude oil feedstock in a petroleum testing laboratory. Using the characterization (assay) data, refineries can estimate the characterization of a crude oil feedstock with respect to hydrocarbon content via a number of methods including use of a probability distribution function for hydrocarbon constituent molecules, as described in U.S. Patent Application No. 13/740,095 (submitted by Applicant).

As global oil supply shifts towards heavy crude oils, refinery feeds will contain an increased amount of trace metals. Creation of efficient demetallation strategies will need an appropriate characterization to identify trace metals. A need for such characterization, therefore, exists.

### SUMMARY OF THE INVENTION

Applicant's claimed invention solves the need for characterization of metal content in a crude oil. In particular, embodiments provide a detailed, molecular-level structural characterization that enables efficient demetellation processes to identify trace metals.

According to the invention, there is provided a computer-implemented method and a system as set out in the appended claims.

In addition, in some embodiments, determining the organometallic class, segment type, and the segment number range includes, for each organometallic class and segment type, identifying a probability distribution function for the organometallic class, segment type, and segment number range, including identifying scale and shape parameters of the probability distribution function. Optionally, the method further includes estimating the metal content and the chemical composition of the given crude oil as a function of the identified probability distribution functions and the relative ratio of each organometallic class, and estimating the physical and chemical properties of the given crude oil as a function of the estimated metal content and chemical composition. Further optionally: (a) the estimated physical properties of the given crude oil include any one of boiling point, density, viscosity, or any combination thereof; (b) the estimated chemical properties of the given crude oil include any one of carbon content, hydrogen content, oxygen content, nitrogen content, sulfur content, vanadium content, nickel content, or any combination thereof; or (c) determining the relative ratio of each organometallic class includes matching the estimated physical and chemical properties of the given crude oil against certain crude oil characterization data in order to determine the relative ratio of each organometallic class in the given crude oil to each other organometallic class that form a chemical composition representative of the given crude oil.

In addition, in some embodiments, the physical property data of the organometallic class includes any one of boiling point, density, and viscosity, or any combination thereof.

In addition, in some embodiments, the chemical property data of the organometallic class includes any one of carbon content, hydrogen content, oxygen content, nitrogen content, sulfur content, vanadium content, nickel content, or any combination thereof.

In addition, in some embodiments, the certain crude oil characterization data includes any one of boiling point, density, viscosity, or any combination thereof. Optionally, the certain crude oil characterization data further includes any one of paraffinic content, naphthenic content, aromatic content, carbon content, hydrogen content, nitrogen content, oxygen content, sulfur content, vanadium content, nickel content, C/H ratio, or any combination thereof.

### BRIEF DESCRIPTION OF THE DRAWINGS

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.

FIG. 1A illustrates an example organometallic class and subclasses thereof, which can be used in embodiments of the present invention.

FIG. 1B illustrates another example organometallic classes and subclasses thereof, which can be used in embodiments of the present invention.

FIG. 2 is a plot of nickel abundance in a given crude oil. The plot illustrates the distribution of nickel porphyrin subclasses as compared to the number of carbon atoms in a sample crude oil. The subclasses are as indicated.

FIG. 3 is a model-predicted true boiling point for a blend made from three crude oils, according to embodiments of the present invention.

FIG. 4 is a model-predicted API gravity for a blend made from three crude oils, according to embodiments of the present invention.

FIG. 5 is schematic view of an example computer network in which embodiments of the present environment may be implemented.

FIG. 6 is a block diagram of the internal structure of a computer in the computer network of FIG. 5.

FIG. 7 is a flow diagram of an example embodiment of a computer implemented method of characterizing a metal content of a given crude oil, according to principles of the present invention.

FIGs. 8A-B are screen views of display content in an example embodiment illustrating a formed characterization of a chemical composition according to principles of the present invention.

DETAILED DESCRIPTION OF THE INVENTION

A description of example embodiments of the invention follows.

Glossary

The term "alkyl," as used herein, refers to a saturated aliphatic branched or straight-chain monovalent hydrocarbon radical having the specified number of carbon atoms. Thus, "$C_1$-$C_6$ alkyl" means a radical having from 1-6 carbon atoms in a linear or branched arrangement. Examples of "$C_1$-$C_6$ alkyl" include , *n*-propyl, *i*-propyl, *n*-butyl, *i*-butyl, *sec*-butyl, *t*-butyl, *n*-pentyl, *n*-hexyl, 2-methylbutyl, 2-methylpentyl, 2-ethylbutyl, 3-methylpentyl, and 4-methylpentyl. Alkyl can be optionally substituted with $C_1$-$C_4$ alkyl, $C_1$-$C_6$ alkoxy, sulfide, and -N($R^1$)($R^2$) wherein $R^1$ and $R^2$ are each independently selected from -H and $C_1$-$C_3$ alkyl.

The term "methyl" and "methylene," as used herein, can also be used to refers to a mono-carbon "alkyl" as described herein. Examples of methyl or methylene include, $-CH_3$, $-CH_2-$,

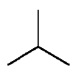, and 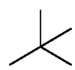.

The term "cycloalkyl", as used herein, means saturated cyclic hydrocarbons, i.e. compounds where all ring atoms are carbons. Examples of cycloalkyl include, but are not limited to, cyclopropyl, cyclobutyl, cyclopentyl, cyclohexyl, and cycloheptyl. In some embodiments, cycloalkyl can optionally be substituted with one or more of hydroxyl, $C_1$-$C_{12}$ alkyl, or $C_1$-$C_{12}$ alkoxy.

The term "alkoxy", as used herein, refers to an "alkyl-O-" group, wherein alkyl is defined above. Examples of alkoxy group include methoxy or ethoxy groups.

The term "amino," as used herein, means an "$-NH_2$," an "$NHR_p$," or an "$NR_pR_q$," group, wherein $R_p$ and $R_q$ may be any of the alkyl, alkenyl, alkynyl, alkoxy, cycloalkyl, cycloalkoxy, aryl, heteroaryl, and bicyclic carbocyclic groups. In the present invention, the amino may be primary ($NH_2$), secondary ($NHR_p$) or tertiary ($NR_pR_q$). A (di)alkylamino group is an instance of an amino group substituted with one or two alkyls.

The term "aryl," as used herein, refers to an aromatic monocyclic or polycyclic (e.g. bicyclic or tricyclic) carbocyclic ring system. Thus, "$C_6$-$C_{18}$ aryl" is a 6-18 membered monocyclic or polycyclic system. Aryl systems include optionally substituted groups such as phenyl, biphenyl, naphthyl, phenanthryl, anthracenyl, or pyrenyl. An aryl can be optionally substituted. Examples of suitable substituents on an aryl include hydroxyl, $C_1$-$C_{12}$ alkyl, $C_6$-$C_{18}$ aryl, and (5-12 atom) heteroaryl,.

In some embodiments, a $C_6$-$C_{18}$ aryl is selected from the group consisting of phenyl, indenyl, naphthyl, azulenyl, heptalenyl, biphenyl, indacenyl, acenaphthylenyl, fluorenyl, phenalenyl, phenanthrenyl, anthracenyl, cyclopentacyclooctenyl or benzocyclooctenyl. In some embodiments, a $C_6$-$C_{18}$ aryl is selected from the group consisting of phenyl, naphthalene, anthracene, 1H-phenalene, tetracene, and pentacene.

The term "carbocyclyl," as used herein, refers to a cyclic group with only ring carbon atoms. "Carbocyclyl" includes 3-12-membered saturated or unsaturated aliphatic cyclic hydrocarbon rings or 6-12-membered aryl rings. Carbocyclyls include cycloalkyl and aryl. Carbocyclyl can be a monocyclic or polycyclic ring system, with cycloalkyl rings fused to aryl rings. Carbocyclyl can optionally substituted in the same manner as alkyl or aryl, described herein.

The term "heteroaryl," as used herein, refers to aromatic groups wherein one or more carbon atoms is substituted

with a heteroatom (O, S, or N). A (5-12 atom) heteroaryl group refers to a monocyclic or polycyclic ring system with 5 to 12 atoms, wherein at least one atom is a heteroatom. A heteroaryl group can be monocyclic or polycyclic, e.g. a monocyclic heteroaryl ring fused to one or more carbocyclic aromatic groups or other monocyclic heteroaryl groups. Examples of heteroaryl groups include, but are not limited to, pyridinyl, pyridazinyl, imidazolyl, pyrimidinyl, pyrazolyl, triazolyl, pyrazinyl, quinolyl, isoquinolyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, purinyl, oxadiazolyl, thiazolyl, thiadiazolyl, furazanyl, benzofurazanyl, benzothiophenyl, benzotriazolyl, benzothiazolyl, benzoxazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl, and azaindolyl. Suitable substituents for heteroaryl are as defined above with respect to aryl group.

The term "hetero," as used herein, refers to the replacement of at least one carbon atom member in a ring system with at least one heteroatom selected from N, S, and O. "Hetero" also refers to the replacement of at least one carbon atom member in a cyclic or acyclic system. A hetero ring system or a hetero acyclic system may have 1, 2, or 3 carbon atom members replaced by a heteroatom.

The term "organometallic," as used herein, refers to an organic chemical compound containing at least one bond between a nitrogen atom and a metal.

The term "sulfide," as used herein, refers to $C_1$-$C_{30}$ alkyl-S- or -S-$C_1$-$C_{30}$ alkyl.

Molecular Based Characterization of Metal Content

A molecular structure-based approach to characterizing crude oil and petroleum fractions with respect to hydrocarbon content is presented in U.S. Patent Application No. 13/740,095. The method uses individual hydrocarbon molecules as a vector of incremental structural features that can describe the composition, reactions, and properties of petroleum mixtures. Chemical compositions of the individual hydrocarbon molecules are inferred from analytical results (assay data) of the crude oil. These analytical results provide physical properties such as the boiling point, the density, and the vapor pressure, as well as chemical properties, such as atom content of the crude oil. This methodology allows refineries to estimate the chemical composition of the crude oil, as well as estimate the physical properties such as the boiling point, density, and vapor pressure for both the crude oil and various fractions found at different stages of the refining process.

There remains a need, however, for methods that allow refineries to characterize a metal composition including metal content. A molecular structure-based approach to characterizing crude oil and petroleum fractions that contain metal content is presented herein. Analytical results of crude oil can provide physical properties such as the boiling point, the density, and the vapor pressure, and can also provide chemical properties, such as metal content of the crude oil. The presence of a metal can be identified, for example, by Fourier transform ion cyclotron mass spectrometry (FT-ICR MS) of the crude oil. This data can further be used to determine the organometallic complexes in which the metals are associated.

Metal complexes in crude oils are generally present in porphyrin structures. Examples of porphyrin structures are cholophyll or Heme B. Generally, the porphyrin structure is a planar, cyclic molecule that contains four pyrrolic nitrogens linked with methine bridges (=CH-), wherein the metal atom, located in the center of the molecule is bound to nitrogen atoms. An example porphyrin structure, porphine, is seen in structural formula (I):

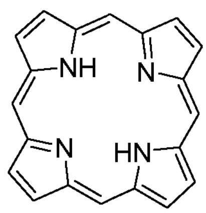

(I)

Substitution of the eight β-hydrogens results in different structures that exist as a homologous series in petroleum. The porphyrin of structural formula (1), therefore, is the simplest porphyrin present in crude oil.

A metal can form a complex with the porphyrin, creating an organometallic species. Many different metals can be coordinated with the porphyrin, including magnesium, iron, nickel, and vanadium. In crude oils, porphyrin structures such as the structure seen in structural formula (I) are often seen coordinated with vanadium and nickel. Accordingly, nickel and vanadium are the most abundant metal types, creating porphryins as seen in example structural formulas (II) and (III):

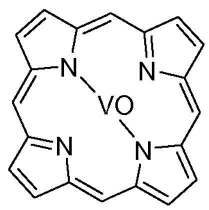

(II)

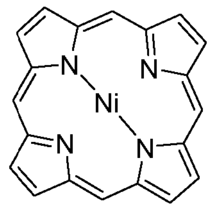

(III)

As used herein, reference to an organometallic "class" refers to an organometallic molecule containing a specific metal. The classes differ with respect to the metal that is bound to the organic compound. For example, the porphyrin of structural formula (II) is directed to one organometallic class, whereas the porphyrin of structural formula (III) is directed to a separate organometallic class. As used herein, an organometallic "subclass" is a core structure within an organometallic class, such as those described below with respect to FIGs. 1A-B . Names for subclasses are as indicated in FIGs. 1A and 1B, which provide prefixes to the term "porphyrin." For example, the indicated "Etio" subclass core structure refers to an "etioporphyrin" core as shown in FIGs. 1A and 1B. The nomenclature in FIGs 1A and 1B is adopted by Applicant for convenience even though it may not be consistent (or entirely consistent) with that used in porphyrin literature (e.g., the "Etio" core structure, as that term is used herein, does not have methyl or ethyl substituents along the ring).

The porphyins in crude oil are not limited merely to the molecules drawn in structural formulas (II)-(III), and FIGs. 1A-B, however. As stated above, the substitution of any one of the eight β-hydrogen results in a different compound within an organometallic class or subclass. The substitution can be, for example, an alkyl or methylene chain optionally substituted with a monocyclic or fused polycyclic carbocyclyl, aryl, or heteroaryl, such as, for example, the compound of structural formulas (IV)-(VI):

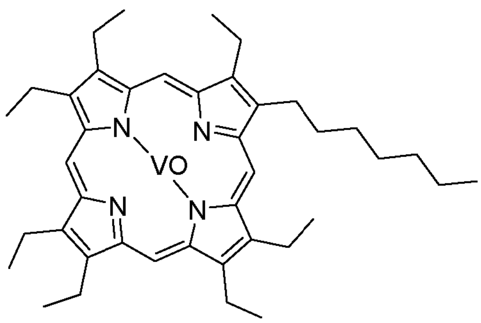

(IV)

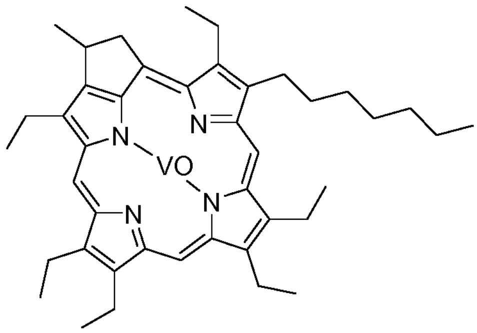

(V)

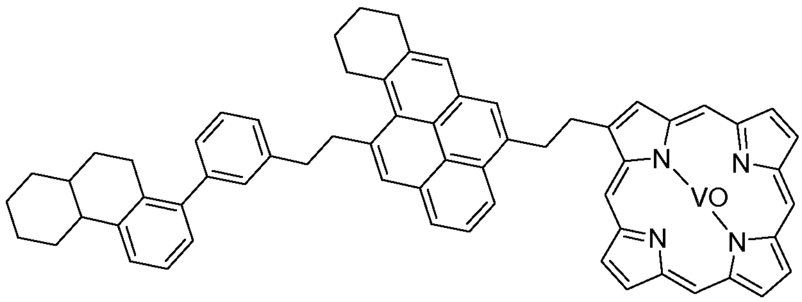

(VI).

The example structural formula (IV) recites use of alkyl linking molecules, thus creating a methylene chain, e.g., -CH2 groups are linked to form an alkyl chain. It should be understood that heteroatoms such as sulfur, nitrogen, or oxygen can be present as well in both the chain and the cyclic moieties. These side chains and cyclic moieties are referred to as "segments" and are described in more detail below.

A crude oil, therefore, contains a mixture of these organometallic classes and subclasses with various substitutions at the eight β-hydrogen positions. To determine the compositions of these organometallic compounds, probability distribution functions are used. To determine the length of the side chain (e.g., a side chain of -CH2 units) and number of other segments (segment frequency) (e.g., of aromatic side rings or naphthenic side rings) bound to each subclass, probability distribution functions are also used. Use of probability distribution functions for each organometallic class and subclass, as well as for the substitutions ("segments," described further below), (a) is less burdensome than traditional methods in terms of the amount of experimental data needed for computations of physical properties of crude oil, and (b) enables easier adjustment of the profile (i.e., composition) of a given crude oil, *e.g.*, to represent drift in properties due to a different depth from which the crude oil is produced. The easier adjustment of the profile is due to the approach in the present invention using the same profile and adjusting the probability distribution functions to match the change in physical and chemical properties accordingly. Use of model compounds created from organometallic classes, subclasses and respective segments, therefore, can be used with the crude oil characterization (assay) data to provide characterization of a given crude oil and to provide estimates of physical and chemical properties of said crude oil in a more robust fashion.

As an example, for the purpose of identifying the probability of the occurrences (hence compositions) of each type of core, a single core structure (e.g., the first structure in FIGs 1A and 1B - namely, the Etio form of the porphyrin complex) can be used as the base from which all other cores can be constructed by adding on segments. In some embodiments, three types of segments can be used: aromatic side ring, naphthenic side ring, and side chain carbon number. The probability of aromatic side ring, can be used to provide a relative composition of rhodo-related subclasses. The probability of naphthenic side rings can give a relative composition of DPEP-related subclasses. These two ring-segments, can allow for creating other cores from the base core. Finally, the side chain carbon number can indicate a chain length - thus allowing molecules (compounds) to be created from cores. Multiplying these probabilities and the class weight can give a composition of each compound. Methods used to compute the composition of each compound from the distribution function and class weight are described in U.S. Patent Publication No. 2013185044, published Jul 18, 2013, entitled "Method of Characterizing Chemical Composition of Crude Oil for Petroleum Processing," U.S. Patent Application No. 13/740,095.

Where the added segment numbers are all zero, then the core structure of the base subclass is obtained. Thus, with respect to the previous paragraph, for example, if the probability of aromatic side rings, naphthenic sides rings, and side chain carbons are all zero, then the core structure of the Etio subclass is obtained, namely etioporphyrin (as shown in FIGs. 1A and 1B). When the probability of side chain carbons is nonzero, this corresponds to additional compounds within the Etio subclass.

Physical and chemical properties of various classes and subclasses of organometallic molecules for crude oil and petroleum fractions are not available to the public and are estimated. Limited data for Porphine were obtained from NIST ThermoData Engine (NIST Standard Reference Database 103b, http://trc.nist.gov/tde.html, accessed 12/7/2015)

Physical properties can also be estimated with the segment-based PC-SAFT equation of state that enables rigor, accuracy, and thermodynamic consistency in the calculation of fundamental physical properties such as vapor pressure, boiling point, density, and heat capacity. See J. Gross, G. Sadowski, Perturbed-Chain SAFT: An Equation-of-State Based on a Perturbation Theory for Chain Molecules, Ind. Eng. Chem. Res. 2001, 40, 1244-1260 (hereinafter "Gross 2001"); J. Gross, O. Spuhl, F. Tumakaka, G. Sadowski, Modeling Copolymer Systems Using the Perturbed-Chain SAFT Equation of State, Ind. Eng. Chem. Res. 2003, 42, 1266-1274 (hereinafter "Gross 2003"). Exact makeup of a given crude oil in terms of various organometallic classes and subclasses can be adjusted so that calculated physical and chemical properties can best match available physical and chemical property measurements of the crude oil, such as metal content (e.g., vanadium content, nickel content, etc.), true boiling point, API gravity, viscosity, C/H ratio, etc. Missing properties of the crude oil assay are then estimated from the chemical compositions and the properties of the organometallic classes and subclasses. Utilities of this approach are further validated with the extensive crude oil assays available to the Applicant. Available references include, for example, Oil & Gas Journal Databook, 2006 Edition, PennWell Corporation, Tulsa, Oklahoma, 2006.

Accordingly, selecting the appropriate organometallic classes and subclasses and practical rules for automatically generating and constructing model molecules from structural segments is key in a molecular structure-based approach to characterizing metal content of crude oil and petroleum fractions. FIG. 1A illustrates twelve example organometallic subclasses (core structures) of the same organometallic class which contain a vanadium atom. FIG. 1B illustrates twelve example organometallic subclasses (core structures) of the same organometallic class which contain a nickel atom. Use of these compounds in the present invention is beneficial as the literature values for the porphyrin compounds without the chelated metal are known.

The physical and chemical properties of these porphyrin structures can then be estimated by PC-SAFT using known or determined parameters for the porphine of structural formula (I) and the desired metal atom (e.g., vanadium or nickel). In an example embodiment, the basic porphyrins of structural formulas (II) and (III) are represented by the porphine of structural formula (I) combined with a metal, such as vanadium or nickel (to create structural formulas (II) and (III), respectively). The PC-SAFT parameters for the porphine of structural formula (I) is determined from experimental

data of the known, physical properties of porphine, including, for example, the boiling point.

The PC-SAFT parameters of a porphine-metal complex (e.g., the compounds of structural formulas (II) and (III)) are determined using available crude assay data for which said metal is available. For example, using the lowest boiling point cut of available crude assay data for which metal content is available, the PC-SAFT parameters for the porphine-metal complex can be determined.

Once the organometallic classes and subclasses are determined, segment type and the segment number range are determined per class/subclass. The segments are bound to the organometallic subclasses (core structures) of the organometallic classes. Given that each of eight different β-hydrogens can be substituted on each organometallic class and subclass, the number of potential model compounds can grow exponentially. To minimize the number of model molecules required to fully characterize physical and chemical properties, the segment number range can be limited. In an example embodiment, the segment number range is between 1 and 60, inclusive. The proper per class/subclass segment type, segment number range, and their respective probability distribution functions can be determined and optimized with the aim to provide a comprehensive and practical representation of diverse physical and chemical properties of crude oil such as metal content (e.g., vanadium content, nickel content, etc.), boiling point, density, viscosity, aromatic content, carbon content, hydrogen content, C/H ratio, sulfur content, nitrogen content, etc.

The segment number range is the range within which the number of each segment types of a class/subclass can be varied. For example, in some embodiments, the 3 segment types related to metal content, and associated number ranges, can be as follows: Aromatic side ring: 0-3; Naphthenic side ring: 0-3; Side chain carbon: 1-60.

As stated above, the porphyrin structures of FIGs. 1A-B can be substituted with various segments. In an example embodiment, the segment type bound to the organometallic classes and subclasses can be a methyl segment, a zero-branch methylene segment, a one-branch methylene segment, a two-branch methylene segment, a carbocyclic segment, a heterocyclic segment, an aryl segment, a heteroaryl segment, a sulfide segment, or any combination thereof.

Examples of these molecular structural repeating units or "segments" that are selected to make up the model hydrocarbon constituent molecules are illustrated below. Here, the $-CH_3$ segment is the methyl end group, the $-CH_2-$ segment is the zero-branch methylene repeat group, the

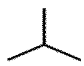

segment is the one-branch methylene group, the

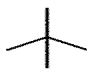

segment is the two-branch methylene group. As defined above, a segment can also be a sulfide or a ring system (*e.g.*, a carbocycle, a heterocycle, an aryl, or a heteroaryl). Examples of ring systems useful in the present invention include:

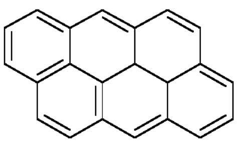 , 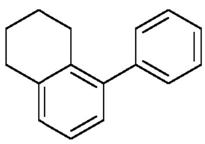 , 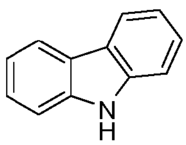 , 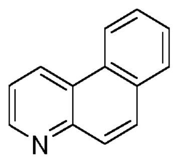 , and 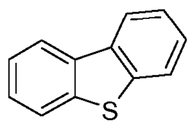 .

Characterization of a Crude Oil or Petroleum Fraction Using a Molecule-Based Approach

To identify the organometallic subclasses of the organometallic class, probability distribution functions and associated parameters for the structural segments are established through systematic investigation. With respect to the metal content, a metal abundance plot obtained from the FT-ICR-MS measurements of the crude or petroleum fractions shows relative abundance of different compounds of an organometallic class as a function of carbon numbers. An example plot for the distribution of nickel porphyrin subclasses can be seen in FIG. 2.

From this data, an algorithm determines the probabilities of segment types and segment number ranges. Example probability distribution functions can include, for example, a gamma distribution function which is a two parameter family of probability distributions of the form

$$p(n) = \frac{(n-L)^{\alpha-1} \cdot e^{-\frac{n-L}{\beta}}}{\beta^{\alpha} \cdot \Gamma(\alpha)} \quad (1)$$

where *p(n)* is the probability of the sample value n; L is the location, the starting point of the probability distribution function; $\alpha$ is the shape factor; $\beta$ is the scale parameter; and $\Gamma(\alpha)$ is the gamma function. The relative contents of the molecules are then identified from regression against assay physical and chemical data such as true boiling point, API gravity, viscosity, metal content, and aromatic content of selected distilled fractions as described below.

The segment-based PC-SAFT equation-of-state (EOS) is used to develop a thermodynamically consistent framework to accurately calculate physical properties for the model hydrocarbon constituent molecules and their mixtures. See Gross 2001; Gross 2003. Segment-based parameters required to characterize the segments that build up the organometallic molecules used to represent metal content of crude oil are thereby obtained for PC-SAFT. These segment-based PC-SAFT parameters include segment ratio, segment size, and segment energy parameters. See Gross 2003. These parameters are identified from regression of experimental data on vapor pressure, liquid density and liquid heat capacity of hundreds of hydrocarbon compounds made up of these segments.

These segment parameters together with the PC-SAFT EOS provide a rigorous and predictive thermodynamic framework based on principles of statistical mechanics. This thermodynamic framework enables systematic exploration of different classes of molecules with various types of segments and segment number ranges. Of particular significance is the unique ability of the PC-SAFT EOS to accurately correlate and predict vapor pressure and liquid density simultaneously. In contrast, typical cubic equations of state such as Peng-Robinson and Redlich-Kwong-Soave are only capable of calculating vapor pressure accurately. These cubic equations of state are incapable of calculating liquid density accurately.

Using the characterization (assay) data, the present invention utilizes an nonlinear regression algorithm to determine the probabilities of segments, *e.g.*, naphthanic side rings, aromatic side rings, methylene side chains, etc., which are then used to calculate compositions of the porphyrin compounds (*e.g.*, the organometallic classes and subclasses together with the segment types and segment number ranges bound to said organometallic classes and subclasses). Use of FT-ICR-MS measurements provide guidance as to the relative abundance of the different compounds of organometallic classes. Therefore, by determining the relative ratio of each organometallic class that forms the chemical composition of the crude oil, a characterization of the crude oil can be obtained. For use of FT-ICR-MS, see Amy M. McKenna, et al. "Unprecedented Ultrahigh Resolution FT-ICR Mass Spectrometry and Parts-Per-Billion Mass Accuracy Enable Direct Characterization of Nickel and Vanadyl Porphyrins in Petroleum from Natural Seeps," Energy Fuels, 28: 2454-2464 (2014). The regression algorithm used in the invention seeks to minimize the sum of squares difference between the characterization (assay) data and the corresponding computed physical and chemical properties that are obtained from the calculated compositions of the compounds and their physical and chemical properties. Based on the sum of squares difference, adjustable parameters are adjusted until the error is smaller than a pre-prescribed tolerance (e.g., 1e-5).

Once characterization (assay) data is received, the system or a user can also "fix" certain variable parameters when creating the composition from the model compounds. Fixing requires certain parameters (e.g., the % by weight of nickel porphyrins, the shape and size of the probability distribution function, etc.) to remain constant, and forces the system to identify a mixture of compounds that conforms to those fixed parameters. This reduces the number of adjustable parameters since metal content data are generally quite limited for a given crude oil. Using a combination of fixed and adjusted parameters, the organometallic and other hydrocarbon compounds in a given crude oil can be determined.

Generating Complete Assays and Properties

Given the molecular distribution parameters for crude oil, various molecular thermodynamic models such as PC-SAFT EOS, group contribution methods, and other methods can be applied to predict a wide variety of physical and chemical properties for crude oil including vapor pressure, density, viscosity, metal content (e.g., vanadium content, nickel content, etc.), paraffin content, naphthene content, aromatic content, carbon content, hydrogen content, C/H ratio, asphaltene content, carbon residue, sulfur content, nitrogen content, total acid number, molecular weight, heat capacity, heating value, heat of vaporization, cloud point, aniline point, wax content, etc. Conversely, if data are available for these physical and chemical properties, they can be used in the regression methodology described above to identify optimal molecular distribution parameters.

The molecular distribution parameters for crude oil further provide the molecular basis for crude oil blending calculations. Traditionally, an arbitrary set of pseudocomponents is applied to all crude oil to be blended, and crude blending is then performed on the pseudocomponent level. In contrast, no such pseudocomponents are required for crude blending with the molecular structure-based approach of the present invention. Here, the chemical compositions of blended crude oil are the sum of the chemical compositions of individual crude oils. Assay properties of a blend can

be rigorously computed based on its molecular representation without the need to go through the artificial step of generating pseudocomponents. FIGs. 3 and 4 show the present invention model-predicted true boiling point and API gravity, respectively, for a blend made from three crude oils. The same methodology described above for modeling crude oil assays and their blends can be applied directly to blending petroleum fractions which have limited boiling point ranges.

Molecular-Based Methodology for Planning, Scheduling, and Process Simulation

As mentioned earlier, crude assays are often used to generate a certain limited number of "pseudocomponents" or "hypothetical components" and their compositions are then used to represent the petroleum mixtures for the purpose of planning, scheduling, and process simulation of petroleum refining processes such as fractionations and reactions. Properties of pseudocomponents are defined to mimic a fraction of the crude assay, i.e., with specific ranges of boiling point and specific gravity. The molecular (and in particular molecular structural) characterization approach offers not only a superior methodology to correlate and predict full assays that subsequently can be used to generate "pseudocomponents," it also offers an important new alternative to this traditional "pseudocomponent" approach for planning, scheduling and process simulation. Specifically, the model molecules and their compositions associated with the molecular distribution parameters can be used directly to represent crude oil and petroleum fractions in planning, scheduling, and process simulation of petroleum refining operations.

Computer Implementation

FIG. 5 illustrates a computer network or similar digital processing environment in which the present invention may be implemented.

Client computer(s)/devices 50 and server computer(s) 60 provide processing, storage, and input/output devices executing application programs and the like. Client computer(s)/devices 50 can also be linked through communications network 70 to other computing devices, including other client devices/processes 50 and server computer(s) 60. Communications network 70 can be part of a remote access network, a global network (e.g., the Internet), a worldwide collection of computers, Local area or Wide area networks, and gateways that currently use respective protocols (TCP/IP, Bluetooth, etc.) to communicate with one another. Other electronic device/computer network architectures are suitable.

FIG. 6 is a diagram of the internal structure of a computer (e.g., client processor/device 50 or server computers 60) in the computer system of FIG. 5. Each computer 50, 60 contains system bus 79, where a bus is a set of hardware lines used for data transfer among the components of a computer or processing system. Bus 79 is essentially a shared conduit that connects different elements of a computer system (e.g., processor, disk storage, memory, input/output ports, network ports, etc.) that enables the transfer of information between the elements. Attached to system bus 79 is I/O device interface 82 for connecting various input and output devices (e.g., keyboard, mouse, displays, printers, speakers, etc.) to the computer 50, 60. Network interface 86 allows the computer to connect to various other devices attached to a network (e.g., network 70 of FIG. 5). Memory 90 provides volatile storage for computer software instructions 92 and data 94 used to implement an embodiment of the present invention (e.g., virtual assay engine, crude oil assay modeler, and supporting code 700 detailed above and below). Disk storage 95 provides nonvolatile storage for computer software instructions 92 and data 94 used to implement an embodiment of the present invention. Central processor unit 84 is also attached to system bus 79 and provides for the execution of computer instructions.

In one embodiment, the processor routines 92 and data 94 are a computer program product (generally referenced 92), including a computer readable medium (e.g., a removable storage medium such as one or more DVD-ROM's, CD-ROM's, diskettes, tapes, etc.) that provides at least a portion of the software instructions for the invention system. Computer program product 92 can be installed by any suitable software installation procedure, as is well known in the art. In another embodiment, at least a portion of the software instructions may also be downloaded over a cable, communication and/or wireless connection. In other embodiments, the invention programs are a computer program propagated signal product 107 embodied on a propagated signal on a propagation medium (e.g., a radio wave, an infrared wave, a laser wave, a sound wave, or an electrical wave propagated over a global network such as the Internet, or other network(s)). Such carrier medium or signals provide at least a portion of the software instructions for the present invention routines/program 92.

In alternate embodiments, the propagated signal is an analog carrier wave or digital signal carried on the propagated medium. For example, the propagated signal may be a digitized signal propagated over a global network (e.g., the Internet), a telecommunications network, or other network. In one embodiment, the propagated signal is a signal that is transmitted over the propagation medium over a period of time, such as the instructions for a software application sent in packets over a network over a period of milliseconds, seconds, minutes, or longer. In another embodiment, the computer readable medium of computer program product 92 is a propagation medium that the computer system 50 may receive and read, such as by receiving the propagation medium and identifying a propagated signal embodied in the propagation medium, as described above for computer program propagated signal product.

Generally speaking, the term "carrier medium" or transient carrier encompasses the foregoing transient signals, propagated signals, propagated medium, storage medium and the like.

FIG. 7 is a flow diagram (processor routine) 700 of an example embodiment of a computer-implemented method of characterizing a metal content of a given crude oil (sample) and updating refinery process in light of the characterized metal content of the crude oil. At 710, the user, computer system 50, 60, or processor 700 provides physical and chemical property data for a plurality of the organometallic class and subclasses and other hydrocarbon classes and subclasses, segment types, and segment number ranges described above. The physical property data of the organometallic classes and subclasses can include, for example, any one of boiling point, density, and viscosity, or any combination thereof. The chemical property data of the organometallic classes and subclasses can include, for example, any one of carbon content, hydrogen content, oxygen content, nitrogen content, sulfur content, vanadium content, nickel content, or any combination thereof.

At 720, computer system 50, 60 or processor receives characterization (assay) data of the given crude oil (sample). As described above this characterization (assay) data can be provided, for example, by ASTM procedures, GC, GC-MS, or FT-ICR-MS, and identify physical properties such as a boiling point, a density, a viscosity, a vapor pressure, or any combination thereof; and chemical properties including the percent weight of paraffinic content, naphthenic content, aromatic content, carbon content, hydrogen content, nitrogen content, oxygen content, sulfur content, metal content (e.g., vanadium content, nickel content), C/H ratio, or any combination thereof.

At 730, using the characterization (assay) data of the given crude oil (sample), and the data of the organometallic classes and subclasses and other hydrocarbon classes and subclasses, the computer system or processor 700 determines the organometallic classes and subclasses and other hydrocarbon classes and subclasses present in the given crude oil. The organometallic classes and subclasses can be selected, for example from the structural formulas represented in FIGs. 1A-B.

At 740, using the characterization (assay) data of the given crude oil, and physical and chemical property data for each segment type and segment number range, the computer system 50, 60 or processor 700 determines the segment types and segment number ranges bound to the organometallic classes and subclasses present in the given crude oil. Step 740 may utilize PC-SAFT to identify the segment types and segment number ranges.

At 750, the computer system 50,60 or processor 700 determines a relative ratio of each organometallic class and subclass to each other organometallic class and subclass that forms a chemical composition representative of the given crude oil (sample). The determined relative ratio and the determined respective organometallic class and subclass, segment type, and segment number range form a characterization of the metal content and the chemical composition of the given crude oil. In an example embodiment, the relative ratio can be determined by a probability distribution function for the organometallic class and subclass, the segment type, and segment number range. Identification of the scale and shape parameters of the probability distribution function can be used to determine the metal content and the chemical composition of the given crude oil.

Using the identified scale and shape parameters, step 750 estimates the metal content and the chemical composition of the given crude oil. Next, step 750 estimates the physical and chemical properties of the given crude oil as a function of the estimated metal content and the chemical composition. In particular, step 750 derives the estimation of the physical and chemical properties, for example, from the known physical and chemical properties for the organometallic classes and subclasses and other hydrocarbon classes and subclasses, segment types, and segment number ranges, as described above. Examples of physical properties that can be estimated include, for example, boiling point, density, viscosity, or any combination thereof. Examples of chemical properties that can be estimated include carbon content, hydrogen content, oxygen content, nitrogen content, sulfur content, vanadium content, nickel content, or any combination thereof.

In another example embodiment, step 750 determines the relative ratio of each organometallic class and subclass by matching the estimated physical and chemical properties of the given crude oil against certain crude oil characterization (assay) data in order to determine the relative ratio of each organometallic class and subclass in the given crude oil to each other organometallic class and subclass that form a chemical composition representative of the given crude oil.

At 760, the computer system 50, 60 or processor 700 compares the estimated physical and chemical properties of the determined chemical composition representative of the crude oil to the actual characterization (assay) data received from, for example, the FT-ICR-MS derived data of the given crude oil. If the estimated data and the characterization (assay) data are consistent to within a pre-prescribed tolerance, step 770 generates a display or graphical representation of the formed characterization of the chemical composition and the metal content. Step 770 provides the generated graphical representation/display as an output to the end user. If the estimated properties are not consistent at step 760, then the processor 700 repeats step 750 to determine (refine) the probability distribution function and relative ratios again until the estimated properties and the characterization (assay) data are consistent to within the pre-prescribed tolerance.

At 780, once the determined chemical composition and metal content are either displayed to the end user (at

770) or determined to be consistent with the characterization (assay) data at 760, the computer system 50,60 can represent (or model), at 780, the given crude oil using the characterized metal content and chemical composition of the given crude oil. This model, including the predicted physical and chemical properties of the given crude oil, at 780, can be used to plan, schedule, simulate, design, optimize, and/or control petroleum refining operations.

In an example embodiment, the present invention characterizes a chemical composition of crude oil, wherein the chemical composition comprises a metal and other hydrocarbon compounds. The resulting characterization is displayed, as output to an end-user, as the formed characterization of the metal content and the chemical composition of the given crude oil, as seen in FIGs. 8A and 8B. FIG 8A displays obtained parameters for the gamma distribution function (see Eq. 1) for the segments of Nickel and Vanadium porphyrins classes. These parameters are used to determine the mass composition of Vanadium porphyrins compounds as shown in FIG 8B. For this particular crude oil, 150 such compounds are identified.

While this invention has been particularly shown and described with references to example embodiments thereof, it will be understood by those skilled in the art that various changes in form and details may be made therein without departing from the scope of the invention encompassed by the appended claims.

For example, computer methods and systems of the present invention describe and predict (by representing a characterization) metal content present in crude oil reactor feedstocks, and reactor products. Embodiments identify individual metal compounds classes, such as but not limited to nickel and vanadyl porphryins, present in crude oil and with such identification enable metal tracking and removal. In one embodiment, the computer-based method is based on molecular-level representation of petroporphyrins and porphyrin-related compounds using assay data of crude oils. Porphyrin-related compounds comprise a porphyrin structure connected to other polycyclic aromatic hydrocarbon structures by methylene or sulfur linkages. The metal content characterization (molecular composition distribution in terms of model compounds of petroporphyrins and porphyrin-related classes) is used in crude oil processing models to track metal content through the distillation process and to predict make-up of reactor products.

## Claims

**1.** A computer-implemented method (700) of characterizing a chemical composition of crude oil, said method comprising:

providing physical and chemical property data of a plurality of organometallic classes and subclasses and other hydrocarbon classes and subclasses, segment types and segment number ranges (710), wherein the physical property data of the organometallic classes and subclasses includes any one of boiling point, density, and viscosity, or any combination thereof and the chemical property data of the organometallic classes and subclasses includes any one of carbon content, hydrogen content, oxygen content, nitrogen content, sulfur content, vanadium content, nickel content, or any combination thereof;

providing crude oil characterization data of a given crude oil (720), wherein the provided crude oil characterization data includes any one of boiling point, density, viscosity, or any combination thereof,

characterizing a metal content of the given crude oil by:

a. determining at least one respective organometallic class and subclass derived from the physical and chemical property data for each of the plurality of organometallic classes and subclasses and other hydrocarbon classes and subclasses and the crude oil characterization data (730), wherein the organometallic class is a porphyrin compound comprising a metal and the organometallic subclass is a core structure within the organometallic class;

b. determining at least one segment type and segment number range of a segment bound to each organometallic subclass (740), wherein determining the organometallic class, segment type, and the segment number range includes, for each organometallic class and segment type, identifying a probability distribution function for the organometallic class, segment type, and segment number range, including identifying scale and shape parameters of the probability distribution function; and

c. determining a relative ratio between each of the at least one organometallic class that forms a chemical composition representative of the given crude oil (750), such that the determined relative ratio, the determined respective organometallic class and subclass, segment type, and segment number range form a characterization of the metal content and the chemical composition of the given crude oil; and

displaying, as output to an end-user, the formed characterization of the metal content and the chemical composition of the given crude oil (770).

2. The method (700) of Claim 1, wherein the segment type includes a methyl segment, a zero-branch methylene segment, a one-branch methylene segment, a two-branch methylene segment, a carbocyclic segment, a heterocyclic segment, an aryl segment, a heteroaryl segment, a sulfide segment, or any combination thereof.

3. The method (700) of Claim 1, further including, for each organometallic class bound to the at least one segment and for the respective segment number range, estimating physical and chemical property values as a function of organometallic class, segment type, and segment number range.

4. The method (700) of Claim 1, further including estimating the metal content and the chemical composition of the given crude oil as a function of the identified probability distribution functions and the relative ratio of each organometallic class, and estimating physical and chemical properties of the given crude oil as a function of the estimated metal content and chemical composition.

5. The method (700) of Claim 4, wherein the physical properties, being estimated, of the given crude oil include any one of boiling point, density, viscosity, or any combination thereof.

6. The method (700) of Claim 4, wherein the chemical properties, being estimated, of the given crude oil include any one of carbon content, hydrogen content, oxygen content, nitrogen content, sulfur content, vanadium content, nickel content, or any combination thereof.

7. The method (700) of Claim 4, wherein determining the relative ratio of each organometallic class includes matching the physical and chemical properties, being estimated, of the given crude oil against the crude oil characterization data in order to determine the relative ratio of each organometallic class in the given crude oil.

8. The method (700) of Claim 1, further comprising, in a computer, predicting physical and chemical properties of the given crude oil by representing the given crude oil using the characterized metal content and chemical composition of the given crude oil and using a molecular thermodynamic model.

9. The method (700) of Claim 1, wherein the segment number range is between 0 and 3 inclusive for aromatic side rings, between 0 and 3 inclusive for naphthenic side rings, and between 1 and 60 inclusive for side chain carbons.

10. A computer-based crude oil refinery process system comprising:

a computer memory (90) storing certain crude oil characterization data of a given crude oil, wherein the crude oil characterization data includes any one of boiling point, density, viscosity, or any combination thereof; and
a processor (84) coupled to the memory (90) and configured to (i) provide physical and chemical property data of a plurality of organometallic classes and subclasses and other hydrocarbon classes and subclasses, and segment types and segment number ranges, and (ii) characterize metal content of the given crude oil,
wherein the processor is configured to characterize the metal content of the given crude oil by:

a. determining at least one respective organometallic class and subclass derived from the physical and chemical property data for each of the plurality of organometallic classes and subclasses and other hydrocarbon classes and subclasses and the crude oil characterization data, wherein the organometallic class is a porphyrin compound comprising a metal and the organometallic subclass is a core structure within the organometallic class;
b. determining at least one segment type and segment number range of a segment bound to each organometallic subclass, wherein determining the organometallic class, segment type, and the segment number range includes, for each organometallic class and segment type, identifying a probability distribution function for the organometallic class, segment type, and segment number range, including identifying scale and shape parameters of the probability distribution function; and
c. determining a relative ratio between each of the at least one organometallic class that forms a chemical composition representative of the given crude oil, such that the determined relative ratio, the determined respective organometallic class and subclass, segment type, and segment number range form a characterization of the metal content and the chemical composition of the given crude oil; and
displaying, as output to an end-user, the formed characterization of the metal content and the chemical composition of the given crude oil,

wherein the physical property data of the organometallic classes and subclasses includes any one of boiling

point, density, and viscosity, or any combination thereof and the chemical property data of the organometallic classes and subclasses includes any one of carbon content, hydrogen content, oxygen content, nitrogen content, sulfur content, vanadium content, nickel content, or any combination thereof.

**Patentansprüche**

1. Computerimplementiertes Verfahren (700) zur Charakterisierung einer chemischen Zusammensetzung von Rohöl, wobei das Verfahren umfasst:

   Bereitstellen von Daten von physikalischen und chemischen Eigenschaften einer Vielzahl von organometallischen Klassen und Unterklassen und anderer Kohlenwasserstoffklassen und -unterklassen, Segmenttypen und Segmentanzahlbereichen (710), wobei die Daten von physikalischen Eigenschaften der organometallischen Klassen und Unterklassen eines von Siedepunkt, Dichte und Viskosität oder eine beliebige Kombination davon enthalten und die Daten von chemischen Eigenschaften der organometallischen Klassen und Unterklassen eines von Kohlenstoffgehalt, Wasserstoffgehalt, Sauerstoffgehalt, Stickstoffgehalt, Schwefelgehalt, Vanadiumgehalt, Nickelgehalt oder eine beliebige Kombination davon enthalten;
   Bereitstellen von Rohölcharakterisierungsdaten eines gegebenen Rohöls (720), wobei die bereitgestellten Rohölcharakterisierungsdaten eines von Siedepunkt, Dichte, Viskosität oder eine beliebige Kombination davon enthalten,
   Charakterisieren eines Metallgehalts des gegebenen Rohöls durch:

   a. Bestimmen wenigstens einer entsprechenden organometallischen Klasse und Unterklasse abgeleitet von den Daten von physikalischen und chemischen Eigenschaften für jede von der Vielzahl von organometallischen Klassen und Unterklassen und anderen Kohlenwasserstoffklassen und -unterklassen und den Rohölcharakterisierungsdaten (730), wobei die organometallische Klasse eine Porphyrinverbindung ist, die ein Metall umfasst, und die organometallische Unterklasse eine Kernstruktur innerhalb der organometallischen Klasse ist;
   b. Bestimmen wenigstens eines Segmenttyps und Segmentanzahlbereichs eines Segments, das an jede organometallische Unterklasse (740) gebunden ist, wobei Bestimmen der organometallischen Klasse, des Segmenttyps und des Segmentanzahlbereichs für jede organometallische Klasse und jeden Segmenttyp Identifizieren einer Wahrscheinlichkeitsverteilungsfunktion für die organometallische Klasse, den Segmenttyp und den Segmentanzahlbereich einschließt, einschließlich Identifizieren von Skalierungs- und Formparametern der Wahrscheinlichkeitsverteilungsfunktion; und
   c. Bestimmen eines relativen Verhältnisses zwischen jeder von der wenigstens einen organometallischen Klasse, die eine chemische Zusammensetzung bildet, die für das gegebene Rohöl (750) repräsentativ ist, so dass das bestimmte relative Verhältnis, die/der bestimmte entsprechende organometallische Klasse und Unterklasse, Segmenttyp und Segmentanzahlbereich eine Charakterisierung des Metallgehalts und der chemischen Zusammensetzung des gegebenen Rohöls bilden; und

   als Ausgabe für einen Endanwender Anzeigen der gebildeten Charakterisierung des Metallgehalts und der chemischen Zusammensetzung des gegebenen Rohöls (770).

2. Verfahren (700) gemäß Anspruch 1, wobei der Segmenttyp ein Methylsegment, ein unverzweigtes Methylensegment, ein einfach verzweigtes Methylensegment, ein zweifach verzweigtes Methylensegment, ein carbocyclisches Segment, ein heterocyclisches Segment, ein Arylsegment, ein Heteroarylsegment, ein Sulfidsegment oder eine beliebige Kombination davon einschließt.

3. Verfahren (700) gemäß Anspruch 1, ferner enthaltend für jede organometallische Klasse, die an das wenigstens eine Segment gebunden ist, und für den entsprechenden Segmentanzahlbereich Abschätzen von Werten von physikalischen und chemischen Eigenschaften als Funktion von organometallischer Klasse, Segmenttyp und Segmentanzahlbereich.

4. Verfahren (700) gemäß Anspruch 1, ferner enthaltend Abschätzen des Metallgehalts und der chemischen Zusammensetzung des gegebenen Rohöls als Funktion der identifizierten Wahrscheinlichkeitsverteilungsfunktionen und des relativen Verhältnisses jeder organometallischen Klasse und Abschätzen von physikalischen und chemischen Eigenschaften des gegebenen Rohöls als Funktion des/der abgeschätzten Metallgehalts und chemischen Zusammensetzung.

5. Verfahren (700) gemäß Anspruch 4, wobei die abgeschätzten physikalischen Eigenschaften des gegebenen Rohöls eines von Siedepunkt, Dichte, Viskosität oder eine beliebige Kombination davon enthalten.

6. Verfahren (700) gemäß Anspruch 4, wobei die abgeschätzten chemischen Eigenschaften des gegebenen Rohöls eines von Kohlenstoffgehalt, Wasserstoffgehalt, Sauerstoffgehalt, Stickstoffgehalt, Schwefelgehalt, Vanadiumgehalt, Nickelgehalt oder eine beliebige Kombination davon enthalten.

7. Verfahren (700) gemäß Anspruch 4, wobei Bestimmen des relativen Verhältnisses jeder organometallischen Klasse Abgleichen der abgeschätzten physikalischen und chemischen Eigenschaften des gegebenen Rohöls mit den Rohölcharakterisierungsdaten einschließt, um das relative Verhältnis jeder organometallischen Klasse in dem gegebenen Rohöl zu bestimmen.

8. Verfahren (700) gemäß Anspruch 1, ferner umfassend Vorhersagen von physikalischen und chemischen Eigenschaften des gegebenen Rohöls in einem Computer durch Darstellen des gegebenen Rohöls unter Verwendung des/der charakterisierten Metallgehalts und chemischen Zusammensetzung des gegebenen Rohöls und Verwenden eines molekularen thermodynamischen Modells.

9. Verfahren (700) gemäß Anspruch 1, wobei der Segmentanzahlbereich zwischen einschließlich 0 und 3 für aromatische Seitenringe, zwischen einschließlich 0 und 3 für naphthenische Seitenringe und zwischen einschließlich 1 und 60 für Seitenketten-Kohlenstoffe beträgt.

10. Computerunterstütztes Rohölraffinerie-Verfahrenssystem umfassend:

einen Computerspeicher (90), der bestimmte Rohölcharakterisierungsdaten eines gegebenen Rohöls speichert, wobei die Rohölcharakterisierungsdaten eines von Siedepunkt, Dichte, Viskosität oder eine beliebige Kombination davon enthalten; und
einen Prozessor (84), der an den Speicher (90) gekoppelt ist und dafür gestaltet ist, (i) Daten von physikalischen und chemischen Eigenschaften einer Vielzahl von organometallischen Klassen und Unterklassen und anderen Kohlenwasserstoffklassen und -unterklassen und Segmenttypen und Segmentanzahlbereichen bereitzustellen, und (ii) Metallgehalt des gegebenen Rohöls zu charakterisieren,
wobei der Prozessor dafür gestaltet ist, den Metallgehalt des gegebenen Rohöls zu charakterisieren durch:

a. Bestimmen wenigstens einer organometallischen Klasse und Unterklasse abgeleitet von den Daten von physikalischen und chemischen Eigenschaften für jede von der Vielzahl von organometallischen Klassen und Unterklassen und anderen Kohlenwasserstoffklassen und - unterklassen und den Rohölcharakterisierungsdaten, wobei die organometallische Klasse eine Porphyrinverbindung ist, die ein Metall umfasst, und die organometallische Unterklasse eine Kernstruktur innerhalb der organometallischen Klasse ist;
b. Bestimmen wenigstens eines Segmenttyps und Segmentanzahlbereichs eines Segments, das an jede organometallische Unterklasse gebunden ist, wobei Bestimmen der organometallischen Klasse, des Segmenttyps und des Segmentanzahlbereichs für jede organometallische Klasse und jeden Segmenttyp Identifizieren einer Wahrscheinlichkeitsverteilungsfunktion für die organometallische Klasse, den Segmenttyp und den Segmentanzahlbereich einschließt, einschließlich Identifizieren von Skalierungs- und Formparametern der Wahrscheinlichkeitsverteilungsfunktion; und
c. Bestimmen eines relativen Verhältnisses zwischen jeder von der wenigstens einen organometallischen Klasse, die eine chemische Zusammensetzung bildet, die für das gegebene Rohöl repräsentativ ist, so dass das bestimmte relative Verhältnis, die/der bestimmte entsprechende organometallische Klasse und Unterklasse, Segmenttyp und Segmentanzahlbereich eine Charakterisierung des Metallgehalts und der chemischen Zusammensetzung des gegebenen Rohöls bilden; und

als Ausgabe für einen Endanwender Anzeigen der gebildeten Charakterisierung des Metallgehalts und der chemischen Zusammensetzung des gegebenen Rohöls,
wobei die Daten von physikalischen Eigenschaften der organometallischen Klassen und Unterklassen eines von Siedepunkt, Dichte und Viskosität oder eine beliebige Kombination davon enthalten und die Daten von chemischen Eigenschaften der organometallischen Klassen und Unterklassen eines von Kohlenstoffgehalt, Wasserstoffgehalt, Sauerstoffgehalt, Stickstoffgehalt, Schwefelgehalt, Vanadiumgehalt, Nickelgehalt oder eine beliebige Kombination davon enthalten.

## Revendications

1. Procédé mis en œuvre par ordinateur (700) pour caractériser une composition chimique de pétrole brut, ledit procédé comprenant :

   la fourniture de données de propriétés physiques et chimiques d'une pluralité de classes et sous-classes organométalliques et d'autres classes et sous-classes d'hydrocarbures, de types de segments et de plages de numéros de segments (710), les données de propriétés physiques des classes et sous-classes organométalliques comprenant l'un quelconque parmi le point d'ébullition, la densité et la viscosité, ou toute combinaison de ceux-ci, et les données de propriétés chimiques des classes et sous-classes organométalliques comprenant l'un quelconque parmi la teneur en carbone, la teneur en hydrogène, la teneur en oxygène, la teneur en azote, la teneur en soufre, la teneur en vanadium, la teneur en nickel, ou toute combinaison de celles-ci ;
   la fourniture de données de caractérisation de pétrole brut d'un pétrole brut donné (720), dans lequel les données de caractérisation de pétrole brut fournies incluent l'un quelconque parmi le point d'ébullition, la densité, la viscosité ou toute combinaison de ceux-ci,
   la caractérisation d'une teneur en métaux du pétrole brut donné par :

   a. détermination d'au moins une classe et sous-classe organométalliques respectives dérivées des données de propriétés physiques et chimiques pour chacune de la pluralité de classes et sous-classes organométalliques et d'autres classes et sous-classes d'hydrocarbures et des données de caractérisation de pétrole brut (730), la classe organométallique étant un composé de porphyrine comprenant un métal et la sous-classe organométallique étant une structure centrale au sein de la classe organométallique ;
   b. détermination d'au moins un type de segment et d'une plage de numéros de segment d'un segment lié à chaque sous-classe organométallique (740), la détermination de la classe organométallique, du type de segment et de la plage de numéros de segment comprenant, pour chaque classe organométallique et type de segment, l'identification d'une fonction de distribution de probabilité pour la classe organométallique, le type de segment et la plage de numéros de segment, y compris l'identification des paramètres d'échelle et de forme de la fonction de distribution de probabilité ; et
   c. détermination d'un rapport relatif entre chacune de la ou des classes organométalliques qui forme une composition chimique représentative du pétrole brut donné (750), de telle sorte que le rapport relatif déterminé, la classe et la sous-classe organométalliques respectives déterminées, le type de segment et la plage de numéros de segment forment une caractérisation de la teneur en métaux et de la composition chimique du pétrole brut donné ; et

   l'affichage, comme sortie pour un utilisateur final, de la caractérisation formée de la teneur en métaux et de la composition chimique du pétrole brut donné (770).

2. Procédé (700) selon la revendication 1, dans lequel le type de segment comprend un segment méthyle, un segment méthylène à zéro branche, un segment méthylène à une branche, un segment méthylène à deux branches, un segment carbocyclique, un segment hétérocyclique, un segment aryle, un segment hétéroaryle, un segment sulfure ou toute combinaison de ceux-ci.

3. Procédé (700) selon la revendication 1, comprenant en outre, pour chaque classe organométallique liée à l'au moins un segment et pour la plage de numéros de segment respective, l'estimation de valeurs de propriétés physiques et chimiques en fonction de la classe organométallique, du type de segment et de la plage de numéros de segment.

4. Procédé (700) selon la revendication 1, comprenant en outre l'estimation de la teneur en métaux et de la composition chimique du pétrole brut donné en fonction des fonctions de distribution de probabilité identifiées et du rapport relatif de chaque classe organométallique, et l'estimation des propriétés physiques et chimiques du pétrole brut donné en fonction de la teneur en métaux et de la composition chimique estimées.

5. Procédé (700) selon la revendication 4, dans lequel les propriétés physiques, étant estimées, du pétrole brut donné comprennent l'un quelconque parmi le point d'ébullition, la densité, la viscosité ou toute combinaison de ceux-ci.

6. Procédé (700) selon la revendication 4, dans lequel les propriétés chimiques, étant estimées, du pétrole brut donné comprennent l'une quelconque parmi la teneur en carbone, la teneur en hydrogène, la teneur en oxygène, la teneur en azote, la teneur en soufre, la teneur en vanadium, la teneur en nickel, ou toute combinaison de celles-ci.

**7.** Procédé (700) selon la revendication 4, dans lequel la détermination du rapport relatif de chaque classe organométallique comprend la correspondance des propriétés physiques et chimiques, estimées, du pétrole brut donné avec les données de caractérisation du pétrole brut afin de déterminer le rapport relatif de chaque classe organométallique dans le pétrole brut donné.

**8.** Procédé (700) selon la revendication 1, comprenant en outre, dans un ordinateur, la prédiction des propriétés physiques et chimiques du pétrole brut donné en représentant le pétrole brut donné en utilisant la teneur en métaux **caractérisée** et la composition chimique du pétrole brut donné et en utilisant un modèle thermodynamique moléculaire.

**9.** Procédé (700) selon la revendication 1, dans lequel la plage de numéros de segments est comprise entre 0 et 3 inclus pour les cycles latéraux aromatiques, entre 0 et 3 inclus pour les cycles latéraux naphténiques, et entre 1 et 60 inclus pour les carbones de chaîne latérale.

**10.** Système de traitement de raffinage de pétrole brut basé sur un ordinateur comprenant :

une mémoire informatique (90) stockant certaines données de caractérisation de pétrole brut d'un pétrole brut donné, les données de caractérisation de pétrole brut comprenant l'un quelconque parmi le point d'ébullition, la densité, la viscosité ou toute combinaison de ceux-ci ; et
un processeur (84) couplé à la mémoire (90) et configuré pour (i) fournir des données de propriétés physiques et chimiques d'une pluralité de classes et sous-classes organométalliques et d'autres classes et sous-classes d'hydrocarbures, et des types de segments et des plages de numéros de segments, et (ii) caractériser la teneur en métaux du pétrole brut donné,
dans lequel le processeur est configuré pour caractériser la teneur en métaux du pétrole brut donné par :

a. détermination d'au moins une classe et une sous-classe organométalliques respectives dérivées des données de propriétés physiques et chimiques pour chacune de la pluralité de classes et sous-classes organométalliques et d'autres classes et sous-classes d'hydrocarbures et des données de caractérisation du pétrole brut, la classe organométallique étant un composé de porphyrine comprenant un métal et la sous-classe organométallique étant une structure centrale au sein de la classe organométallique ;
b. détermination d'au moins un type de segment et une plage de numéros de segment d'un segment lié à chaque sous-classe organométallique, la détermination de la classe organométallique, du type de segment et de la plage de numéros de segment comprenant, pour chaque classe organométallique et type de segment, l'identification d'une fonction de distribution de probabilité pour la classe organométallique, le type de segment et la plage de numéros de segment, y compris l'identification des paramètres d'échelle et de forme de la fonction de distribution de probabilité ; et
c. détermination d'un rapport relatif entre chacune de la ou des classes organométalliques qui forment une composition chimique représentative du pétrole brut donné, de telle sorte que le rapport relatif déterminé, la classe et sous-classe organométalliques respectives déterminées, le type de segment et la plage de numéros de segment forment une caractérisation de la teneur en métaux et de la composition chimique du pétrole brut donné ; et

l'affichage, comme sortie pour un utilisateur final, de la caractérisation formée de la teneur en métaux et de la composition chimique du pétrole brut donné,
dans lequel les données de propriétés physiques des classes et sous-classes organométalliques comprennent l'un quelconque parmi le point d'ébullition, la densité et la viscosité, ou toute combinaison de ceux-ci, et les données de propriétés chimiques des classes et sous-classes organométalliques comprennent l'une quelconque parmi la teneur en carbone, la teneur en hydrogène, la teneur en oxygène, la teneur en azote, la teneur en soufre, la teneur en vanadium, la teneur en nickel ou toute combinaison de celles-ci.

Etio

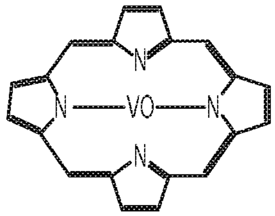

Rhodo-Etio

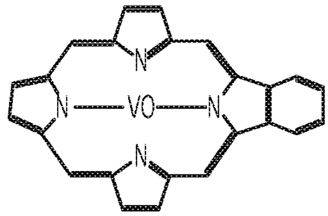

Di-Rhodo

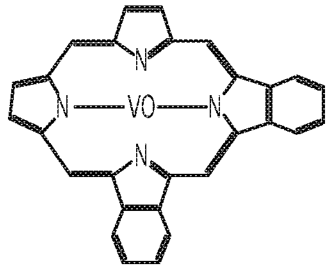

Tri-Rhodo

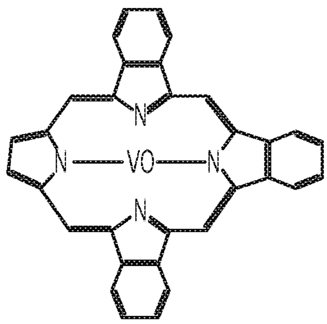

DPEP

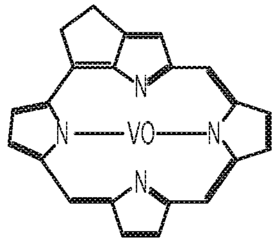

Rhodo-DPEP

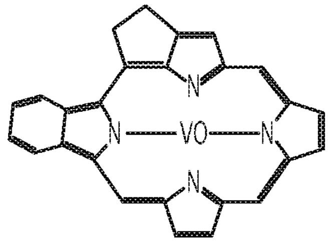

Di-Rhodo-DPEP

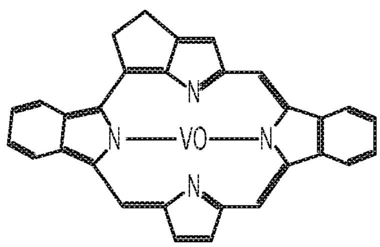

Tri-Rhodo-DPEP

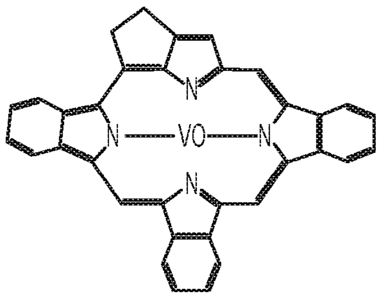

Di-DPEP

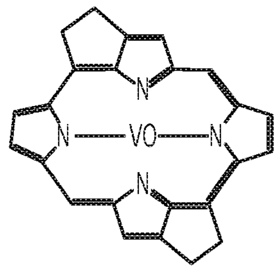

Rhodo-Di-DPEP

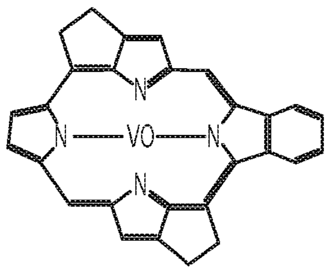

Di-Rhodo-Di-DPEP

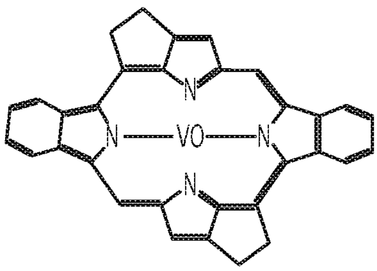

Tri-Rhodo-Di-DPEP

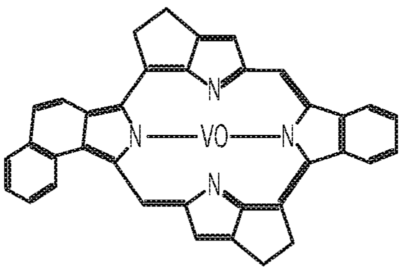

FIG. 1A

Etio

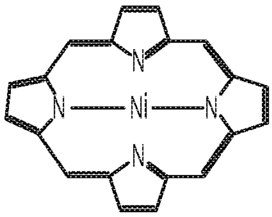

Rhodo-Etio

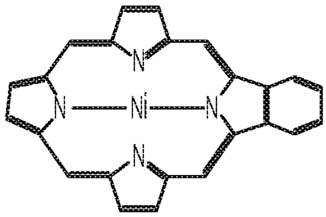

Di-Rhodo

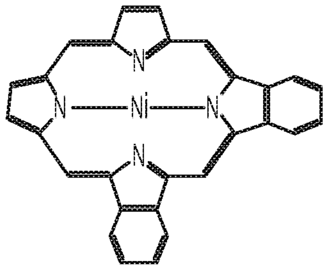

Tri-Rhodo

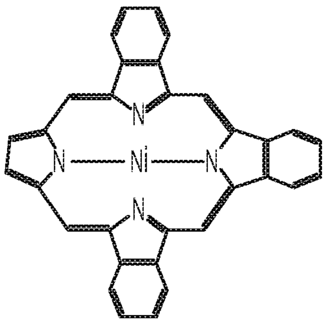

DPEP

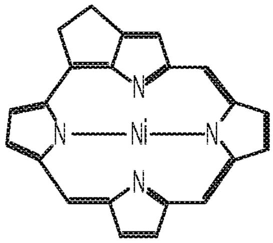

Rhodo-DPEP

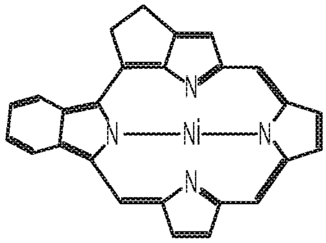

Di-Rhodo-DPEP

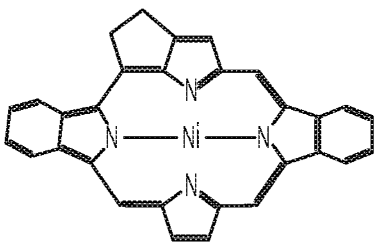

Tri-Rhodo-DPEP

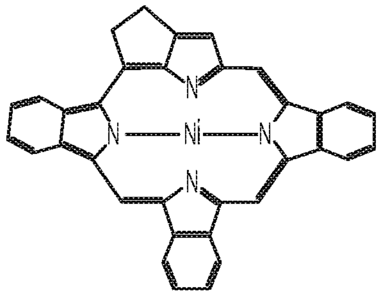

Di-DPEP

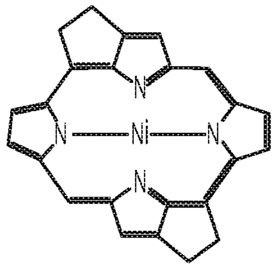

Rhodo-Di-DPEP

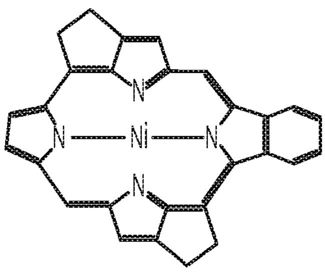

Di-Rhodo-Di-DPEP

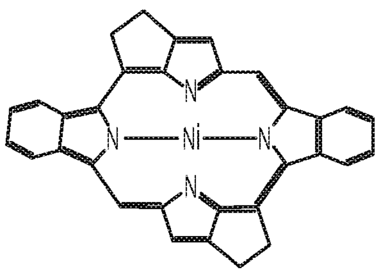

Tri-Rhodo-Di-DPEP

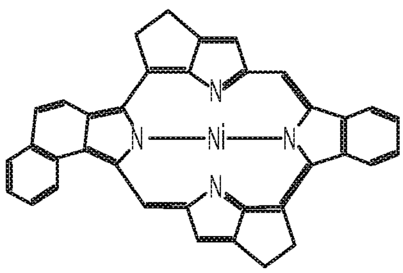

FIG. 1B

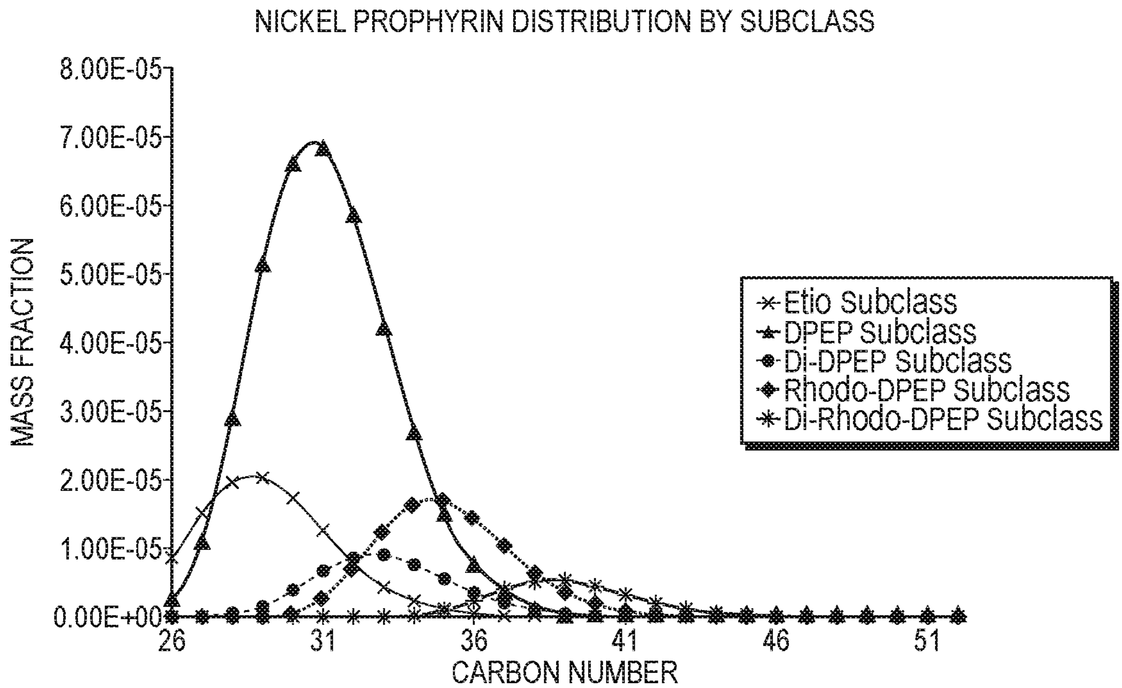

NICKEL PROPHYRIN DISTRIBUTION BY SUBCLASS
MASS FRACTION
8.00E-05
7.00E-05
6.00E-05
5.00E-05
4.00E-05
3.00E-05
2.00E-05
1.00E-05
0.00E+00
26
31
36
41
46
51
CARBON NUMBER
Etio Subclass
DPEP Subclass
Di-DPEP Subclass
Rhodo-DPEP Subclass
Di-Rhodo-DPEP Subclass


FIG. 2

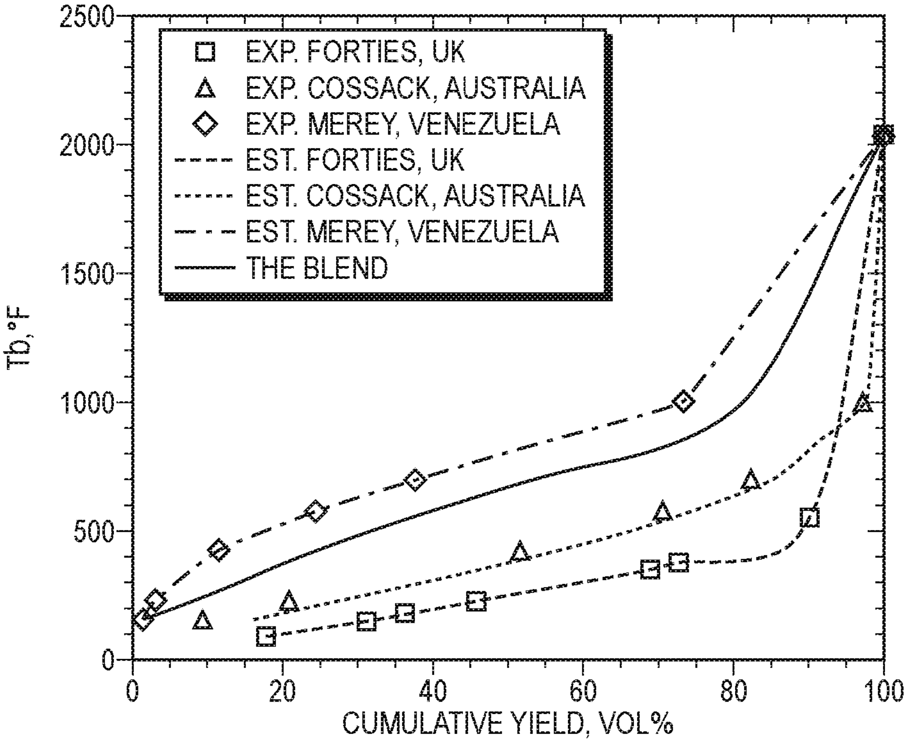

2500
2000
1500
1000
500
0
Tb,°F
EXP. FORTIES, UK
EXP. COSSACK, AUSTRALIA
EXP. MEREY, VENEZUELA
EST. FORTIES, UK
EST. COSSACK, AUSTRALIA
EST. MEREY, VENEZUELA
THE BLEND
0
20
40
60
80
100
CUMULATIVE YIELD, VOL%


FIG. 3

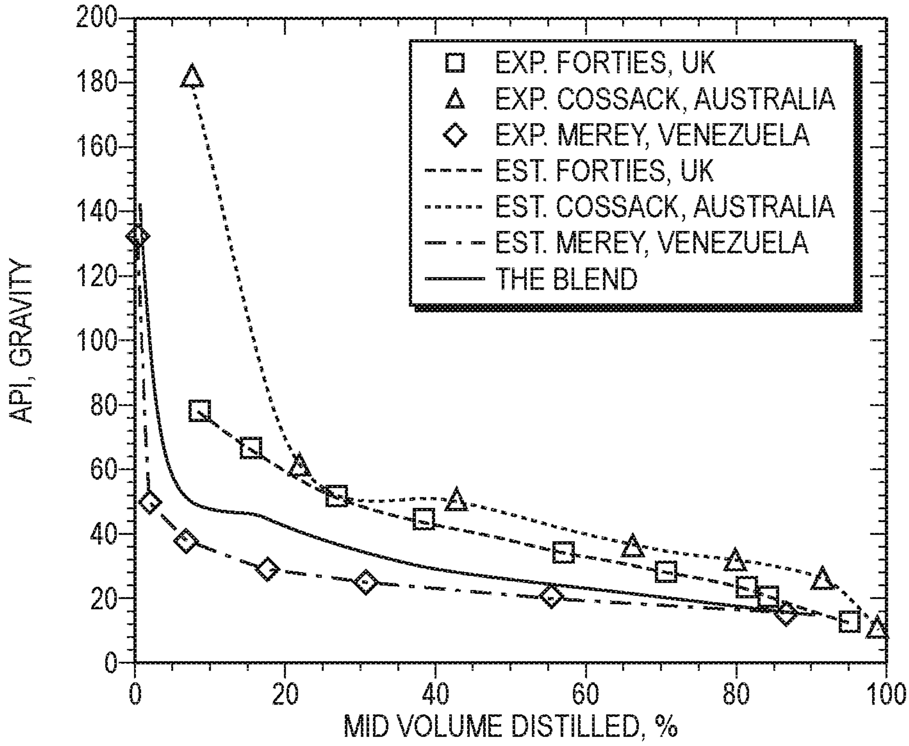

200
180
160
140
120
100
80
60
40
20
0
API, GRAVITY
0
20
40
60
80
100
MID VOLUME DISTILLED, %
EXP. FORTIES, UK
EXP. COSSACK, AUSTRALIA
EXP. MEREY, VENEZUELA
EST. FORTIES, UK
EST. COSSACK, AUSTRALIA
EST. MEREY, VENEZUELA
THE BLEND


FIG. 4

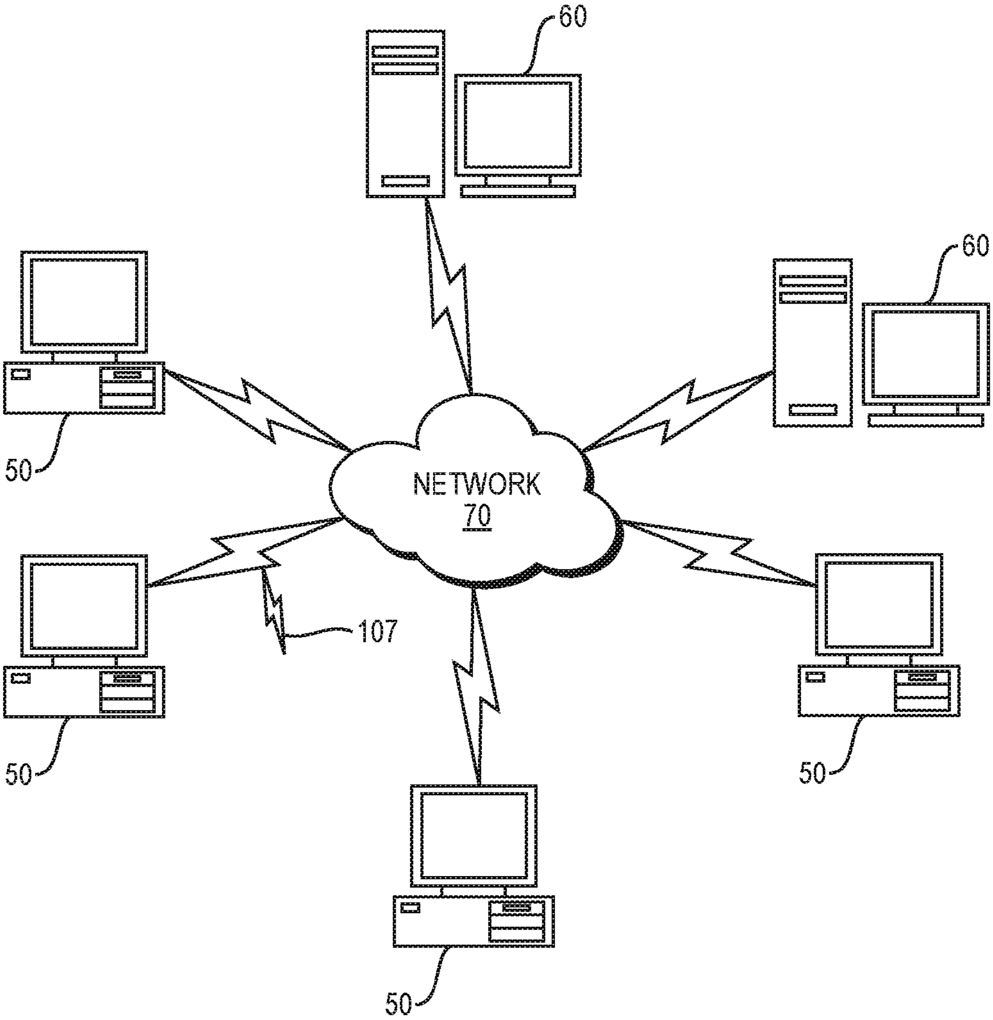


FIG. 5

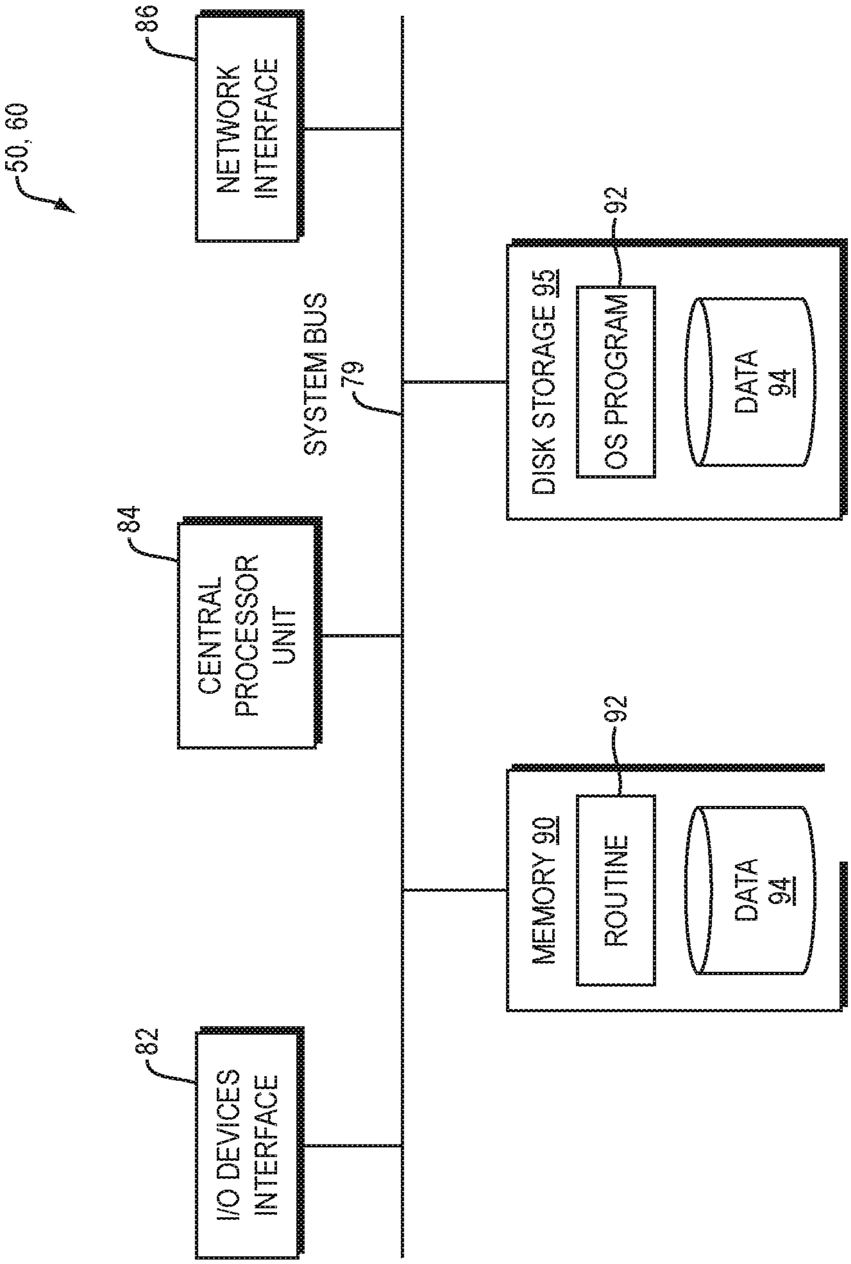

50, 60
NETWORK INTERFACE
86
CENTRAL PROCESSOR UNIT
84
I/O DEVICES INTERFACE
82
SYSTEM BUS
79
DISK STORAGE 95
OS PROGRAM
92
DATA
94
MEMORY 90
ROUTINE
92
DATA
94


FIG. 6

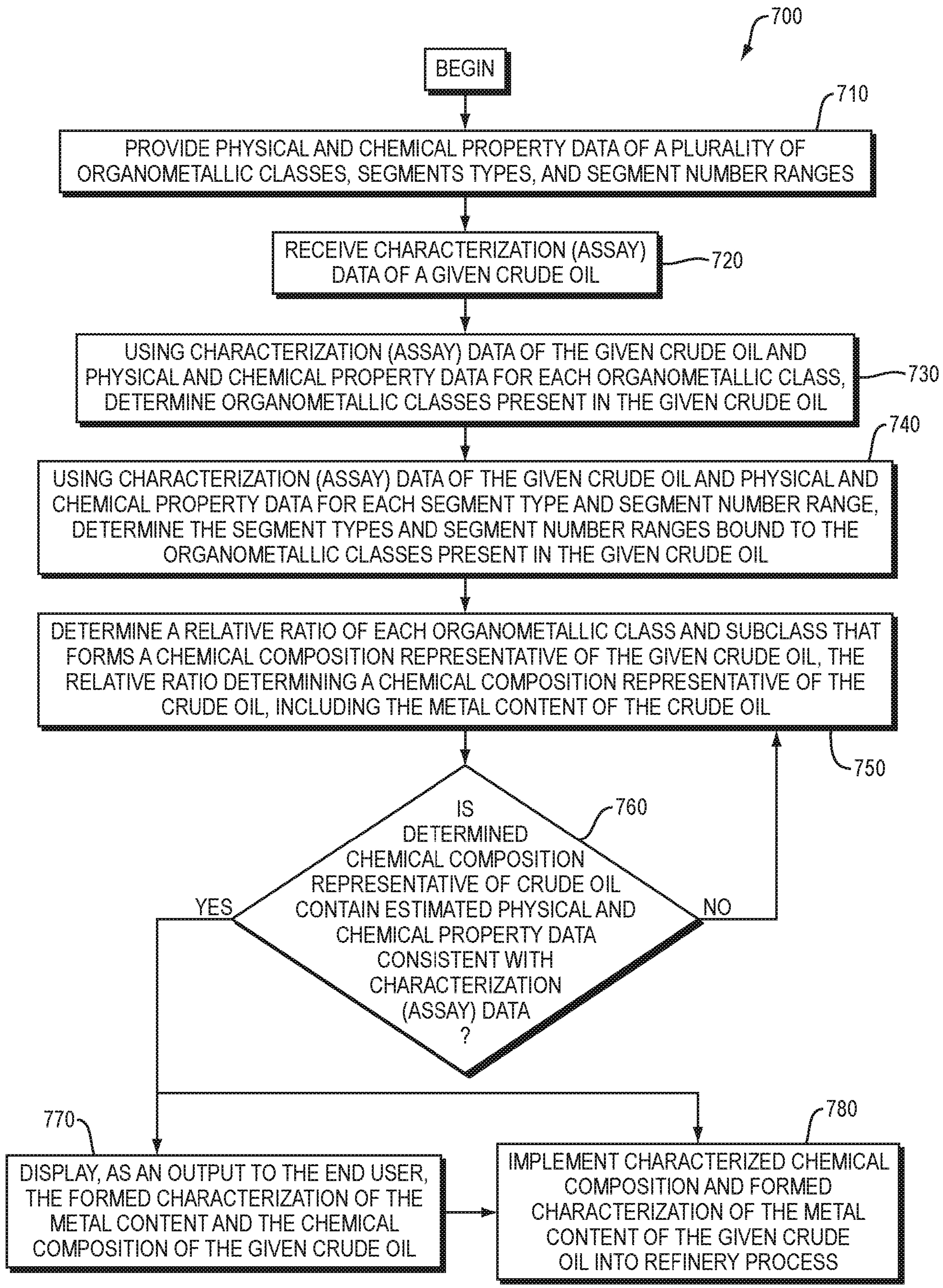
700
BEGIN
710
PROVIDE PHYSICAL AND CHEMICAL PROPERTY DATA OF A PLURALITY OF ORGANOMETALLIC CLASSES, SEGMENTS TYPES, AND SEGMENT NUMBER RANGES
720
RECEIVE CHARACTERIZATION (ASSAY) DATA OF A GIVEN CRUDE OIL
730
USING CHARACTERIZATION (ASSAY) DATA OF THE GIVEN CRUDE OIL AND PHYSICAL AND CHEMICAL PROPERTY DATA FOR EACH ORGANOMETALLIC CLASS, DETERMINE ORGANOMETALLIC CLASSES PRESENT IN THE GIVEN CRUDE OIL
740
USING CHARACTERIZATION (ASSAY) DATA OF THE GIVEN CRUDE OIL AND PHYSICAL AND CHEMICAL PROPERTY DATA FOR EACH SEGMENT TYPE AND SEGMENT NUMBER RANGE, DETERMINE THE SEGMENT TYPES AND SEGMENT NUMBER RANGES BOUND TO THE ORGANOMETALLIC CLASSES PRESENT IN THE GIVEN CRUDE OIL
DETERMINE A RELATIVE RATIO OF EACH ORGANOMETALLIC CLASS AND SUBCLASS THAT FORMS A CHEMICAL COMPOSITION REPRESENTATIVE OF THE GIVEN CRUDE OIL, THE RELATIVE RATIO DETERMINING A CHEMICAL COMPOSITION REPRESENTATIVE OF THE CRUDE OIL, INCLUDING THE METAL CONTENT OF THE CRUDE OIL
750
760
IS DETERMINED CHEMICAL COMPOSITION REPRESENTATIVE OF CRUDE OIL CONTAIN ESTIMATED PHYSICAL AND CHEMICAL PROPERTY DATA CONSISTENT WITH CHARACTERIZATION (ASSAY) DATA ?
YES
NO
770
DISPLAY, AS AN OUTPUT TO THE END USER, THE FORMED CHARACTERIZATION OF THE METAL CONTENT AND THE CHEMICAL COMPOSITION OF THE GIVEN CRUDE OIL
780
IMPLEMENT CHARACTERIZED CHEMICAL COMPOSITION AND FORMED CHARACTERIZATION OF THE METAL CONTENT OF THE GIVEN CRUDE OIL INTO REFINERY PROCESS

FIG. 7

| Molecular Class | Wt(%) | Carbon Number | CentroidTb (F) | ρ(API) | Segment Name | Segment Structure | Function | β | α |
|---|---|---|---|---|---|---|---|---|---|
| **Porphyrin molecules** | | | | | | | | | |
| Nickel porphyrins | 0.142 | 37 | 585.37 | 971.48 | Aromatic side ring | 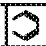 | Gamma | **1.15** | **0.1** |
| | | | | | Naphthenic side ring | 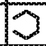 | Gamma | **0.15** | **12.3** |
| | | | | | Side chain carbon number | $-C_nH_{2n+1}$ | Gamma | 0.391872 | 37.7768 |
| Vanadium porphyrins | 0.043 | 34 | 575.13 | 990.77 | Aromatic side ring | 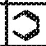 | Gamma | **1.15** | **0.1** |
| | | | | | Naphthenic side ring | 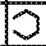 | Gamma | **0.15** | **12.3** |
| | | | | | Side chain carbon number | $-C_nH_{2n+1}$ | Gamma | 0.115478 | 100 |

FIG. 8A

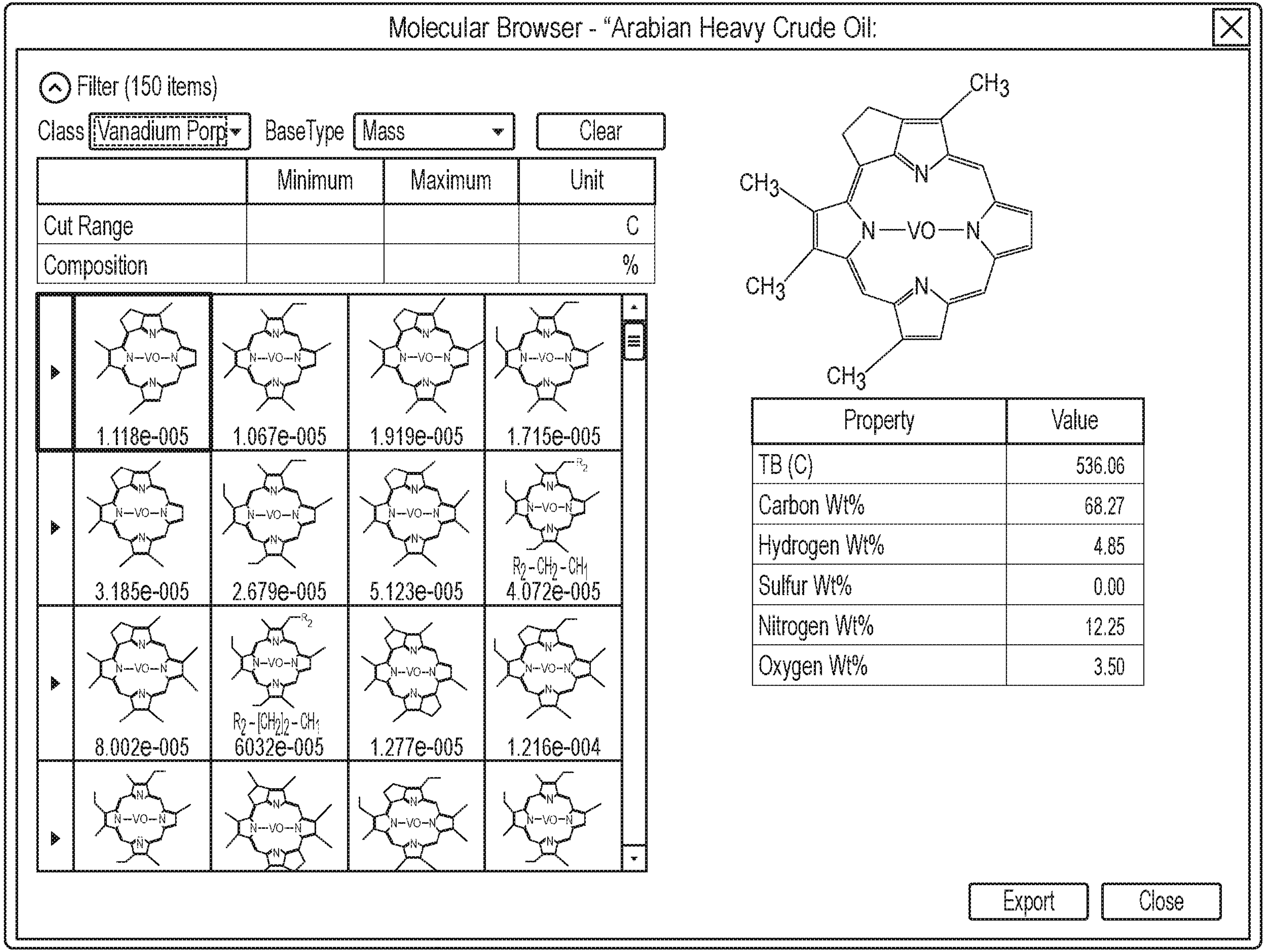
Molecular Browser - "Arabian Heavy Crude Oil:
Filter (150 items)
Class Vanadium Porp
BaseType Mass
Clear
Minimum
Maximum
Unit
Cut Range
C
Composition
%
1.118e-005
1.067e-005
1.919e-005
1.715e-005
3.185e-005
2.679e-005
5.123e-005
4.072e-005
8.002e-005
6032e-005
1.277e-005
1.216e-004
Property
Value
TB (C)
536.06
Carbon Wt%
68.27
Hydrogen Wt%
4.85
Sulfur Wt%
0.00
Nitrogen Wt%
12.25
Oxygen Wt%
3.50
Export
Close

FIG. 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 740095
- US 2013185044 A
- US 740095 A


### Non-patent literature cited in the description


- **J. GROSS ; G. SADOWSKI.** Perturbed-Chain SAFT: An Equation-of-State Based on a Perturbation Theory for Chain Molecules. *Ind. Eng. Chem. Res.,* 2001, vol. 40, 1244-1260
- **J. GROSS ; O. SPUHL ; F. TUMAKAKA ; G. SADOWSKI.** Modeling Copolymer Systems Using the Perturbed-Chain SAFT Equation of State. *Ind. Eng. Chem. Res.,* 2003, vol. 42, 1266-1274
- Oil & Gas Journal Databook. PennWell Corporation, 2006
- **AMY M. MCKENNA et al.** Unprecedented Ultrahigh Resolution FT-ICR Mass Spectrometry and Parts-Per-Billion Mass Accuracy Enable Direct Characterization of Nickel and Vanadyl Porphyrins in Petroleum from Natural Seeps. *Energy Fuels,* 2014, vol. 28, 2454-2464